# EUROPÄISCHE PATENTSCHRIFT

(11) **EP 1 310 476 B1**
(45) Veröffentlichungstag und Bekanntmachung des Hinweises auf die Patenterteilung: **28.09.2005**
(21) Anmeldenummer: 02024496.8
(22) Anmeldetag: 30.10.2002
(51) Int. Cl.: C07C 45/52, C07C 49/12

(54) **Verfahren zur Herstellung von 2,3-Pentandion**
Process for the synthesis of 2,3-Pentandione
Procédé de préparation de 2,3-Pentandione

(30) Priorität: 12.11.2001 DE 10155553
(43) Veröffentlichungstag der Anmeldung: 14.05.2003
(73) Patentinhaber: Symrise GmbH & Co. KG, 37603 Holzminden (DE)
(72) Erfinder: Lambrecht, Stefan, Dr., 37603 Holzminden (DE); Franke, Oliver, 37671 Höxter (DE); Zahlmann, Klaus, 37603 Holzminden (DE)
(74) Vertreter: Eisenführ, Speiser & Partner

(56) Entgegenhaltungen:
- EP-A- 0 983 987
- US-A- 2 799 707
- DATABASE CROSSFIRE BEILSTEIN [Online] Beilstein Institut zur Förderung der chemischen Wissenschaften, Frankfurt am Main, DE; Database accession no. 643575 XP002230743 & NEUBERG ET AL.: BIOCHEM. Z., Bd. 275, 1935, Seite 342

## Beschreibung

Die Erfindung betrifft ein neues Herstellungsverfahren für 2,3-Pentandion ausgehend von Hydroxyaceton und Paraldehyd.

2,3-Pentandion ist ein wichtiger Aromastoff (Allured's Flavor and Fragrance Materials - 1995, Allured Publishing Corporation, 1995, S. 22). Zudem ist 2,3-Pentandion ein wichtiger Rohstoff zur Herstellung alkylsubstituierter Pyrazine (DE 100 22 361 C1).

Die Herstellung von 2,3-Pentandion ist an sich bekannt. Die Herstellung erfolgt beispielsweise ausgehend von Hydroxyaceton und Paraldehyd in einem einstufigen Prozess und kann durch das folgende Schema verdeutlicht werden:

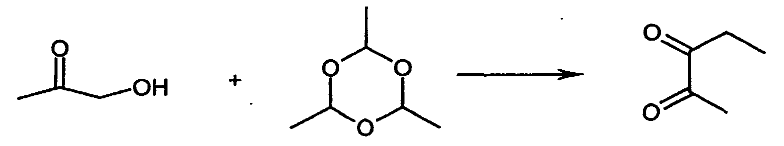

Das bekannte Syntheseverfahren (US-A 2 799 707) ergibt das gewünschte 2,3-Pentandion aus Hydroxyaceton und Paraldehyd in einem unwirtschaftlichen Verfahren. Die schlechte Ausbeute an 2,3-Pentandion von 48 % und die lange Reaktionszeit (ca. 60 Stunden) sind besonders nachteilig.

Ein Verfahren, das auf einfache Weise und in guter Ausbeute 2,3-Pentandion liefert, ist deshalb von großem Interesse.

Gegenstand der vorliegenden Erfindung ist daher ein Verfahren zur Herstellung von 2,3-Pentandion aus Hydroxyaceton und Paraldehyd, dadurch gekennzeichnet, dass die Umsetzung in Gegenwart einer wässrigen Phase enthaltend eine starke organische oder anorganische Säure mit einem pKs-Wert von kleiner oder gleich 4 in Gegenwart eines Phasentransferkatalysators durchgeführt wird.

Es wurde gefunden, dass durch die Zugabe eines Phasentransferkatalysators im Vergleich zum Stand der Technik (US-A 2 799 707) die Ausbeute stark verbessert werden kann. Zudem konnte die Reaktionszeit deutlich verkürzt werden und die Raum-Zeit-Ausbeute deutlich gesteigert werden.

Bevorzugt ist die Zugabe eines Dosiergemisches enthaltend Hydroxyaceton und Paraldehyd sowie gegebenenfalls ein Verdünnungsmittel zu einer wässrigen Lösung enthaltend eine Säure mit einem pKs-Wert von kleiner oder gleich 4 und einen Phasentransferkatalysator.

Geeignete Phasentransferkatalysatoren für das erfindungsgemäße Verfahren sind z.B. Phosphonium-, Sulfonium- und Ammoniumverbindungen. Bevorzugt sind Ammoniumverbindungen. Bevorzugte Ammoniumverbindungen entsprechen der Formel

R¹R²R³R⁴N⁺X⁻

- wobei R¹ bis R⁴: - unabhängig voneinander- C-1 bis C-18-Alkyl oder Benzyl
und
- X⁻: Iodid, Chlorid, Hydroxid oder Hydmgensulfat
bedeuten. Besonders bevorzugte Phasentransferkatalysatoren sind die Tetrabutylammoniumsalze (R¹ bis R⁴ = Butyl).

Erfindungsgemäße Säuren mit einem pKs-Wert, der kleiner oder gleich 4 ist, können anorganische und organische Säuren sein. Besonders geeignet sind Säuren mit einem pKs-Wert von kleiner oder gleich 1. Bevorzugt sind anorganische Säuren. Besonders bevorzugt sind HCl, HBr, Schwefelsäure und Salpetersäure.

Die Menge an Säure liegt bei 5 bis 50 Gew.-% bezogen auf die Menge der wässrigen Phase, bevorzugt bei 10 bis 30 Gew.-% bezogen auf die Menge der wässrigen Phase.

Typischerweise liegt die Menge an Paraldehyd bezogen auf eingesetztes Hydroxyaceton bei 40 bis 200 Gew.-%, bevorzugt bei 50 bis 100 Gew.-%.

Typischerweise liegt die Menge an Paraldehyd im Dosiergemisch bei 40 bis 200 Gew.-% bezogen auf die Menge des Hydroxyacetons, bevorzugt bei 50 bis 100 Gew.-%.

Die Dosierung des Dosiergemisches erfolgt bei einer Reaktionstemperatur (Sumpftemperatur) im Bereich von 35 bis 100°C, bevorzugt bei 40 bis 75°C, besonders bevorzugt bei 45 bis 60°C. Die Temperatur des Dosiergemisches liegt üblicherweise im Bereich von 0 bis 40°C, bevorzugt im Bereich von 10 bis 30°C. Die Dosierdauer beträgt üblicherweise 1 bis 30 Stunden, bevorzugt 5 bis 20 Stunden.

Das erfindungsgemäße Verfahren kann in Gegenwart von Verdünnungsmitteln durchgeführt werden. Bevorzugt wird auf die Zugabe von inerten organischen Verdünnungsmitteln verzichtet. Bevorzugtes Verdünnungsmittel ist Wasser.

Nach Beendigung der Dosierung des Dosiergemisches kann sich eine Nachreaktionszeit anschließen, bevorzugt ist eine Nachreaktionszeit im Bereich von 0,05 bis 3 Stunden, besonders bevorzugt im Bereich von 0,1 bis 1 Stunde.

In einer weiteren bevorzugten Ausführungsform schließt sich nach Beendigung der Dosierung direkt die Destillation des Gemisches enthaltend Wasser und 2,3-Pentandion an. Diese Destillation kann unter Normaldruck oder unter Vakuum durchgeführt werden. Weiterhin kann diese Destillation auch mit überhitztem Wasserdampf, der in den Reaktor eingeleitet wird, durchgeführt werden. Üblicherweise liegt der Druck des Wasserdampfes im Bereich von 3 bis 10 bar.

Bevorzugt ist eine Wasserdampfdestillation bei Normaldruck durch Zusatz von Wasser.

Die chemische Ausbeute liegt typischerweise um 85 % des im Unterschuss eingesetzten Eduktes, wodurch eine wirtschaftliche Herstellung von 2,3-Pentandion möglich wird.

Nach dem erfindungsgemäßen Verfahren kann unter schonenden Reaktionsbedingungen ohne Verwendung aufwendiger Spezialapparaturen aus industriell verfügbaren Ausgangsmaterialien 2,3-Pentandion in guter Ausbeute hergestellt werden. Das erfindungsgemäße Verfahren eignet sich daher besonders zur wirtschaftlichen Herstellung von 2,3-Pentandion im technischen und industriellen Maßstab.

Das erfindungsgemäße Verfahren kann beispielsweise folgendermaßen durchgeführt werden:

Ein Dosiergemisch enthaltend Hydroxyaceton und Paraldehyd sowie gegebenenfalls ein Verdünnungsmittel, wird zu einer wässrigen Lösung enthaltend eine Säure mit einem pKs-Wert von kleiner oder gleich 4 und einen Phasentransferkatalysator zugetropft. Die Reaktionstemperatur wird weitgehend konstant gehalten, sie liegt üblicherweise im Bereich von 35 bis 100°C.

Nach dem Ende der Dosierung und gegebenenfalls einer Nachreaktionszeit wird Wasser zugegeben und ein Gemisch, das im Wesentlichen aus Wasser und 2,3-Pentandion besteht, aus dem Reaktionsgemisch abdestilliert. Nach Abschluss der Destillation wird das 2,3-Pentandion vom Wasser abgetrennt. Die Reinheit des so erhaltenen 2,3-Pentandions liegt bereits bei 85 bis 95 Gew.-%. Eine weitere Reinigung kann bei Bedarf beispielsweise durch eine weitere Destillation erfolgen.

### Beispiel 1

### Herstellung von 2,3-Pentandion im Labormaßstab:

### Herstellung des Dosiergemisches:

100 g Hydroxyaceton und 84 g Paraldehyd werden gemischt.

### Durchführung der Reaktion:

27 g konzentrierte Salzsäure, 33 g Wasser und 2,5 g Tetrabutylammoniumbromid-Lsg., 50 gew.-%ig in Wasser, werden zusammengegeben und auf 50°C erhitzt. Bei dieser Temperatur wird das Dosiergemisch (Temperatur Dosiergemisch: 19 - 21°C) innerhalb von 7 Stunden eindosiert.

Dann werden weitere 38 g Wasser zu dem Reaktionsgemisch gegeben und ein Gemisch, das im Wesentlichen aus Wasser und 2,3-Pentandion besteht, bei Normaldruck abdestilliert. Anschließend werden die Phasen getrennt. Man erhält 125 g 2,3-Pentandion, 92 gew.-%ig laut Gaschromatogramm. Dies entspricht einer Ausbeute von 85 % des im Unterschuss eingesetzten Eduktes.

### Beispiel 2

### Herstellung von 2,3-Pentandion im technischen Maßstab

### Herstellung des Dosiergemisches:

150 kg Hydroxyaceton und 126 kg Paraldehyd werden gemischt.

### Durchführung der Reaktion:

40 kg konzentrierte Salzsäure, 50 kg Wasser und 4 kg Tetrabutylammoniumbromid-Lsg., 50 gew.-%ig in Wasser, werden nacheinander in den Reaktor gefüllt und auf 50°C erhitzt. Bei dieser Temperatur wird das Dosiergemisch (Temperatur Dosiergemisch: 10°C) innerhalb von 10 Stunden eindosiert.

Dann werden weitere 60 kg Wasser zu dem Reaktionsgemisch gegeben und ein Wasser/2,3-Pentandion-Gemisch bei Normaldruck abdestilliert. Anschließend werden die Phasen getrennt. Man erhält 174 kg 2,3-Pentandion, 94 gew.-%ig laut Gaschromatogramm. Dies entspricht einer Ausbeute von 81 % des im Unterschuss eingesetzten Eduktes.

## Patentansprüche

1. Verfahren zur Herstellung von 2,3-Pentandion aus Hydroxyaceton und Paraldehyd, **dadurch gekennzeichnet, dass** die Umsetzung in Gegenwart einer wässrigen Phase enthaltend eine starke organische oder anorganische Säure mit einem pKs-Wert von kleiner oder gleich 4 in Gegenwart eines Phasentransferkatalysators durchgeführt wird.

2. Verfahren nach Anspruch 1, **dadurch gekennzeichnet, dass** als Phasentransferkatalysatoren Phosphonium-, Sulfonium- oder Ammoniumverbindungen verwendet werden.

3. Verfahren nach einem der Ansprüche 1 oder 2, **dadurch gekennzeichnet, dass** die Säuremenge 5 bis 50 Gew.-% bezogen auf die Menge der wässrigen Phase beträgt.

4. Verfahren nach mindestens einem der Ansprüche 1 bis 3, **dadurch gekennzeichnet, dass** eine anorganische Säure mit einem pKs-Wert von kleiner oder gleich 1 verwendet wird.

5. Verfahren nach mindestens einem der Ansprüche 1 bis 4, **dadurch gekennzeichnet, dass** die Menge an Paraldehyd bei 40 bis 200 Gew.-% bezogen auf die Menge an eingesetztem Hydroxyaceton liegt.

6. Verfahren nach einem der Ansprüche 1 bis 5, **dadurch gekennzeichnet, dass** die Dosierung bei einer Reaktionstemperatur von 35°C bis 100°C erfolgt.

## Claims

1. Process for the production of 2,3-pentanedione from hydroxyacetone and paraldehyde, **characterised in that** the reaction is performed in the presence of an aqueous phase containing a strong organic or inorganic acid having a pKa value of less than or equal to 4 and in the presence of a phase transfer catalyst.

2. Process according to claim 1, **characterised in that** phosphonium, sulfonium or ammonium compounds are used as phase transfer catalysts.

3. Process according to one of claims 1 or 2, **characterised in that** the amount of acid is 5 to 50 wt.%, relative to the amount of the aqueous phase.

4. Process according to at least one of claims 1 to 3, **characterised in that** an inorganic acid having a pKa value of less than or equal to 1 is used.

5. Process according to at least one of claims 1 to 4, **characterised in that** the amount of paraldehyde is 40 to 200 wt.%, relative to the amount of hydroxyacetone used.

6. Process according to one of claims 1 to 5, **characterised in that** metering takes place at a reaction temperature of 35°C to 100°C.

## Revendications

1. Procédé pour la préparation de 2,3-pentanedione à partir d'hydroxyacétone et de paraldéhyde, **caractérisé en ce que** l'on réalise la réaction en présence d'une phase aqueuse contenant un acide fort organique ou inorganique avec une valeur pKs inférieure ou égale à 4 en présence d'un catalyseur de transfert de phase.

2. Procédé selon la revendication 1, **caractérisé en ce que** l'on utilise comme catalyseurs de transfert de phase des composés de phosphonium, de sulfonium ou d'ammonium.

3. Procédé selon l'une quelconque des revendications 1 ou 2, **caractérisé en ce que** la quantité d'acide est comprise entre 5 et 50 % en poids rapportée à la quantité de la phase aqueuse.

4. Procédé selon au moins l'une quelconque des revendications 1 à 3, **caractérisé en ce que** l'on utilise un acide inorganique avec une valeur pKs inférieure ou égale à 1.

5. Procédé selon au moins l'une quelconque des revendications 1 à 4, **caractérisé en ce que** la quantité de paraldéhyde est comprise entre 40 et 200 % en poids rapportée à la quantité d'hydroxyacétone utilisée.

6. Procédé selon l'une quelconque des revendications 1 à 5, **caractérisé en ce que** le dosage est réalisé à une température de réaction de 35°C à 100°C.
